**Europäisches Patentamt**

**European Patent Office**

**Office européen des brevets**

(11) Publication number: **0 235 905**
**B1**

# EUROPEAN PATENT SPECIFICATION

(45) Date of publication of patent specification: **19.12.90**

(21) Application number: **87300585.4**

(22) Date of filing: **23.01.87**

(51) Int. Cl.⁵: **A 61 M 5/31**, A 61 M 5/315

(54) Adaptor for injection syringe.

(30) Priority: **23.01.86 IQ 1886**

(43) Date of publication of application:
**09.09.87 Bulletin 87/37**

(45) Publication of the grant of the patent:
**19.12.90 Bulletin 90/51**

(84) Designated Contracting States:
**DE FR GB IT**

(56) References cited:
**DE-C- 361 789**
**GB-A- 180 753**
**US-A-3 063 449**

(73) Proprietor: **Al-Rawi, Omar Mahmood Atia**
**Ameria Al-Firdous quarter No 8/4/630**
**Baghdad (IQ)**

(72) Inventor: **Al-Rawi, Omar Mahmood Atia**
**Ameria Al-Firdous quarter No 8/4/630**
**Baghdad (IQ)**

(74) Representative: **Silverman, Warren et al**
**HASELTINE LAKE & CO. Hazlitt House 28**
**Southampton Buildings Chancery Lane**
**London WC2A 1AT (GB)**

Courier Press, Leamington Spa, England.

## Description

This invention relates to an adaptor for an injection (or hypodermic) syringe for use in particular, but not exclusively, in the injection of insulin from a simple syringe which may be a disposable syringe.

Insulin has been used in the control of diabetes mellitus for many years, especially insofar as the use of bovine insulin is concerned. Currently, there are about 100 million people in the world suffering from diabetes and needing to carry out self medication with insulin preparations. The cost of insulin for use in self medication has become much reduced so that it is rarely a problem insofar as the treatment of patients is concerned. The main problem is that insulin can only be administered by injection and this must be done daily. Another problem arises in respect of the area in which injection is to take place. This should be as large as possible in order to decrease the possibility of rejection and of local side effects of insulin. It is common practice for the patient to be taught to inject himself subcutaneously either in the abdomen or on the front of the thigh. Intramuscular and intravenous injection is also possible, but generally considered to be less suitable for self administration. Many instruments have been invented to help patients inject themselves subcutaneously. These instruments may be of different types and involve injection in different ways. However they all share the same problem of difficulty of control, as well as requiring maintenance and sterilisation. There is a high percentage of failures and the possibility of injecting too large a dose with such instruments, which might be fatal.

It is generally considered that most patients prefer to use simple syringes in carrying out the injection and the use of such syringes has until now been considered to be the safest, cheapest and easiest way of administering insulin. Simple disposable syringes may indeed be used but with these as with injection syringes in general there is the difficulty that controlled administration of insulin can only take place in limited areas of the body such as the abdomen or thigh as already mentioned. One proposal for providing remotely driven operation of an injection syringe disclosed in DE-A-361789 offers a foot-operated drive.

It is an object of the present invention to provide a means for enabling self administration of an injection fluid to be effected by a patient at a wide variety of locations on the body with there being a high degree of control of the instrument during administration.

According to the present invention, there is provided a remote drive adaptor for an injection syringe, the adapter comprising hand held drive means comprising a piston cylinder, whose piston is drivable by hand and on the downward side of which there is a fluid chamber; a plunger housing housing a plunger surmounted by a plunger head drivable within the housing and flexible duct means connecting the fluid chamber of the piston cylinder with the plunger housing for supplying fluid from the piston cylinder to the plunger head to act thereon, the plunger housing being adapted to engage a housing of an injection syringe, with said plunger projecting from its housing to act on a piston rod of the injection syringe to enable fluid powered motion of the plunger to be imparted to the piston rod.

This invention provides a simple device for self use by the patient, enabling the use of a simple syringe as used routinely for injection of insulin but with displacement of the point of application of force by the patient to the piston cylinder of the drive means which acts as a pump providing a hydraulic linkage so that it is fluid power from the pump which is the agent in driving the syringe for injection of insulin.

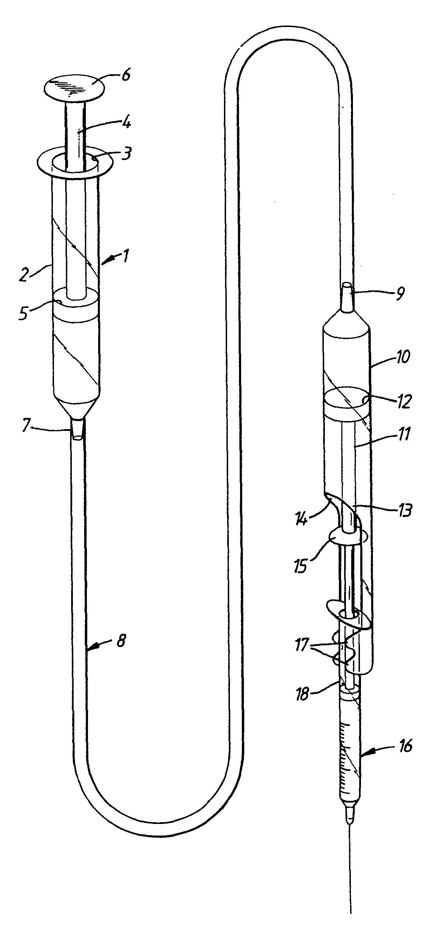

For a better understanding of the invention, and to show how the same can be carried into effect, reference will now be made by way of example only to the accompanying drawing which shows schematically an adaptor according to the present invention in engagement with a syringe for injection of insulin.

Referring to the drawing, there is shown a pump 1 comprising a piston cylinder 2 having an open end 3 through which is inserted a piston 4 whose head 5 is of smaller diameter than the opening 3 and which provides a good seal against the wall of the piston cylinder. The piston carries a handpiece 6 which can be depressed by hand to impart downward displacement to the piston. The lower part of the piston cylinder 2 tapers to a neck 7 which enters a tube 8 of flexible plastics material whose other end fits over a neck 9 on a plunger housing 10. The plunger housing contains a plunger 11 having a head 12 which is in sealing engagement with the wall of the plunger housing 10 and is attached to a rod 13 which extends out of an opening 14 in the housing to engage the head 15 of the piston of a conventional syringe 16 for injection of insulin. The housing 10 is elongated to form two wings 17 which grip the housing 18 of the insulin syringe.

The adaptor is used in the following manner. The flexible tube 8 is connected up at one end to the piston cylinder and at the other end to the plunger housing 10. The plunger 11 is pushed up the plunger housing until it is adjacent the position of connection with the tube 8. With the piston 4 absent from the piston cylinder 2, fluid such as water is introduced into the piston cylinder 2 and allowed to pass through the tube 8 to extend as far as the plunger 11. The level of fluid in the piston cylinder 2 should be such as to provide a quantity of fluid largely equal to the space within the plunger housing 10 below the plunger. With the piston 4 replaced in its housing, the adaptor is ready for use.

A conventional syringe filled with an injectable fluid which in the preferred case will be an insulin preparation is then attached to the adaptor. The wings 17 of the housing 10 are sufficiently flexible to allow them to wrap around and grip the housing 18 of the insulin syringe. The location at which the housing 16 is gripped is such that the top of the head 13 of the syringe will lie immediately below the plunger 11. Indeed the base of the plunger may be shaped as shown to engage the head 15. Then, with the housing 16 of the syringe held in the one hand, which will be assumed hereinafter to be the right hand of a right handed patient, and the piston cylinder 2 held in the other, the needle is injected into the body at a suitable location for injection of the insulin. The right-handed person who has been holding the syringe housing 18 in his right hand will have full control of the syringe at this stage. While the patient is still holding the syringe in the right hand, he then depressed the head 5 of the piston 4 with the left hand and causes the fluid in that piston cylinder 2 to be displaced to act on the plunger 11 to displace it downwardly and in turn act on the handpiece 13 whose depression causes operation of the syringe and injection into the body of insulin.

Throughout the aforesaid operation, the syringe is being held steady in the right hand of the patient so that there is no movement of the syringe during introduction of insulin to the body. There will be no shifting of the needle which is a particular problem when the diabetic patient does not have a steady hand because of neuropathy. Moreover, no longer is it necessary for the patient to be restricted to injecting himself in areas of the body which he can see particularly well to ensure steady injection of the syringe needle and reliable holding of the needle in the body during the injection of the insulin. Provided that the patient can in the first instance manage to introduce the needle with his stronger hand, it does not matter that subsequently it would not be convenient for him to attempt to depress directly the piston of the syringe. The pump 1 will be at any convenient location and an orientation selected which will allow easy depression of the piston 4 with the left hand. Thus it will be possible for injection to take place in the deltoid region, the left upper arm of a right-handed patient or various other locations which are generally more suitable locations for introduction of the insulin than the front of the thigh or the abdomen allowing, in particular intravenous or intramuscular injection to take place.

Indeed, by graduating the piston cylinder 2 in a manner matched to graduations on the housing 18 of the insulin syringe, merely by observing the depression of the piston 4 within its housing, it will be possible to see how much insulin is being administered to the body.

Clinical tests utilising the adaptor of the invention have already yielded satisfactory results. The adaptor of the invention is capable of use any number of times although it is desirable for it to be sterilised before reuse. After a while, too, seals are likely to deteriorate. For health reasons, therefore, although making the adaptor more costly, it should be disposable and utilised likewise with disposable syringes. Thus all parts of the adaptor can be made of plastics material. The adaptor can be utilised many times until its flexible tubing becomes hard or there is a tendency to leak. Tests have shown that it may be reliable for use at least ten times and thus a convenient pack to supply to a patient might comprise ten disposable syringes with a single disposable adaptor of this invention.

**Claims**

1. A remote drive adaptor for an injection syringe, the adapter comprising hand held drive means (1) comprising a piston cylinder (2), whose piston (4) is drivable by hand and on the downward side of which there is a fluid chamber; a plunger housing (10) housing a plunger (11) surmounted by a plunger head (12) drivable within the housing (10) and flexible duct means (8) connecting the fluid chamber of the piston cylinder (2) with the plunger housing (10) for supplying fluid from the piston cylinder to the plunger head (12) to act thereon, the plunger housing (10) being adapted to engage a housing (18) of an injection syringe (16), with said plunger (11) projecting from its housing to act on a piston rod (15) of the injection syringe (16) to enable fluid powered motion of the plunger (11) to be imparted to the piston rod (15).

2. An adaptor according to claim 1, wherein the plunger housing (10) comprises an extension thereof formed in a terminal region with wings (17) adapted to grip the housing (18) of an injection syringe (16) inserted therebetween.

3. An adaptor as claimed in claim 2 wherein the plunger (11) extends from its housing (10) to a position intermediate the housing and the wing extension (17) and terminates externally of the housing in a portion adapted to engage the head of a piston rod (15) of an injection syringe.

4. An injection kit for self-administration of insulin to a patient which comprises an adaptor as claimed in any preceding claims and at least one syringe for use therewith, the piston cylinder (2) of the drive means (1) being transparent and graduated volumetrically to match the volumetric graduations on the syringe (s).

**Patentansprüche**

1. Fernbetätigungs-Adapter für eine Injektionsspritze, welcher Adapter umfaßt: eine von Hand gehaltene Betätigungsvorrichtung (1), die einen Kolbenzylinder (2) enthält, dessen Kolben (4) von Hand betätigbar ist und auf dessen abwärtsgerichteter Seite sich eine Flüssigkeitskammer befindet, ein Kolben-Gehäuse (10), das einen Kolben (11) enthält, der an seinem oberen Ende einen Kolbenkopf (12) aufweist, welcher innerhalb des Gehäuses (10) treibbar ist, und einen flexiblen Kanal (8), der die Flüssigkeitskammer des Kolbenzylinders (2) mit dem Kolben-Gehäuse (10) zum Zuführen von Flüssigkeit von dem Kolbenzylinder zu dem Kolben (12) verbindet, um auf diesen einzuwirken, wobei das Kolben-Gehäuse (10) dazu bestimmt ist, mit einem Gehäuse (18) einer Injektionsspritze (16) in Eingriff zu treten, wobei der Kolben (11) aus seinem Gehäuse vorsteht, damit er auf eine Kolbenstange (15) der Injektionsspritze (16) einwirken kann, um zu ermöglichen, daß eine flüssigkeitsgetriebene Bewegung des Kolbens (11) auf die Kolbenstange (15) übertragen wird.

2. Adapter nach Anspruch 1, bei dem das Kolben-Gehäuse (10) eine Verlängerung aufweist, die in einem Endbereich mit Flügeln (17) ausgebildet ist, welche dazu bestimmt sind, das Gehäuse (18) einer Injektionsspritze (16), die zwische diese gesteckt ist, einzuspannen.

3. Adapter nach Anspruch 2, bei dem sich der Kolben (11) von seinem Gehäuse (10) aus zu einer Position zwischen dem Gehäuse und dem Anbau der Flügel (17) erstreckt und ausserhalb des Gehäuses in einem Teil endet, der dazu bestimmt ist, in Eingriff mit dem Kopf einer Kolbenstange (15) einer Injektionsspritze zu treten.

4. Spritzen-Besteck zur Selbstversorgung eines Patienten mit Insulin, welches Besteck umfaßt: einen Adapter nach einem der vorhergehenden Ansprüche und zumindest eine Spritze zur Benutzung mit diesem, wobei der Kolbenzylinder (2) der Betätigungsvorrichtung (1) durchsichtig ist und volumetrisch abgestuft ist, um mit der volumetrischen Abstufung der Spritze (n) zusammenzupassen.

**Revendications**

1. Adaptateur pour commande à distance d'une seringue à injection, cet adaptateur comprenant un moyen de commande (1) que l'on tient à la main et qui comprend un cylindre (2) de piston, dont le piston (4) peut être commandé manuellement et sur le côté aval duquel se trouve une chambre de fluide; un boîtier (10) de plongeur logeant un plongeur (11) surmonté par une tête (12) de plongeur pouvant être entraînée à l'intérieur du boîtier (10) et un conduit flexible (8) raccordant la chambre de fluide du cylindre (2) de piston au boîtier (10) de plongeur pour l'envoi d'un fluide depuis le cylindre de piston jusqu'à la tête (12) de plongeur afin d'agir sur cette dernière, le boîtier (10) de plongeur étant adapté pour être assemblé au corps (18) d'une seringue (16) à injection, ledit plongeur (11) faisant saillie de son boîtier pour agir sur une tige (15) de piston de la seringue (16) à injection afin que le mouvement du plongeur (11) sous l'action du fluide soit communiqué à la tige (15) de piston.

2. Adaptateur selon la revendication 1, dans lequel le boîtier (10) de plongeur comprend, dans sa région terminale, un prolongement muni d'ailettes (17) adaptées pour exercer une prise sur le corps (18) de la seringue (16) à injection inséré entre ces dernières.

3. Adaptateur selon la revendication 2, dans lequel le plongeur (11) s'étend de son boîtier (10) jusqu'à une position située entre ce boîtier et le prolongement d'ailette (17) et se termine à l'extérieur dudit boîtier par une partie adaptée pour porter contre la tête de la tige (15) de piston de la seringue à injection.

4. Nécessaire d'injection pour auto-administration d'insuline à un patient, comprenant un adaptateur selon l'une quelconque des revendications précédentes et au moins une seringue destinée à être utilisée avec ce dernier, le cylindre (2) de piston du moyen de commande (1) étant transparent et gradué en volume de manière à correspondre aux graduations volumétriques de la seringue ou de seringues.